# EUROPÄISCHE PATENTANMELDUNG

(11) **EP 0 425 443 A1**
(43) Veröffentlichungstag der Anmeldung: **02.05.1991**
(21) Anmeldenummer: 90810805.3
(22) Anmeldetag: 19.10.1990
(51) Int. Cl.: A61K 31/17, A61K 31/44, A61K 9/00, A61K 47/22, A61K 47/00

(54) **Injizierbares parasitizides Mittel**

(30) Priorität: 27.10.1989 CH 3898/89
(71) Anmelder: CIBA-GEIGY AG, CH-4002 Basel (CH)
(72) Erfinder: Oechslein, walter, Dr., CH-4106 Therwil (CH); Gehret, Jean-Claude, Dr., CH-4147 Aesch (CH); Hess, Ernst, Dr., CH-4124 Schönenbuch (CH)

(57) **Zusammenfassung**

Parenteral injizierbare Mittel zur Bekämpfung von Parasiten, die 0,1 bis 10 % eines Benzoylphenylharnstoffes als Wirkstoff, 0,1 bis 60 % eines 1-substituierten Azacycloalkan-2-ons, 2 bis 90 % eines physiologisch verträglichen Tensids oder Tensidgemisches und gegebenenfalls als stabilisierende Komponente 0,05 bis 1 % einer Säure oder eines Puffergemisches sowie ad 100 % eines physiologisch verträglichen hydrophilen Lösungsmittels oder Lösungsmittelgemisches oder ein Gemisch von physiologisch verträglichen hydrophilen und lipophilen Lösungsmitteln enthalten.

## Beschreibung

Die vorliegende Erfindung betrifft ein Mittel zur Bekämpfung von tierschädigenden Parasiten, welches parenteral injizierbar ist und als Wirkstoff mindestens einen schwerlöslichen Benzoylphenylharnstoff enthält.

Injizierbare Zubereitungen stellen wegen der sehr genauen Dosierbarkeit auch kleiner Wirkstoffmengen, der leichten Anwendbarkeit und der geringfügigen Beunruhigung der zu behandelnden Tiere, insbesondere grösserer Nutztiere, wie beispielsweise Rinder, Schafe, Pferde und Esel, oftmals die günstigste Applikationsart dar. Bei der Verwendung von schwerlöslichen Substanzen wie insbesondere den Benzoylphenylharnstoffen in Form injizierbarer Formulierungen ergeben sich jedoch Probleme, wenn mit ihnen ein wirksamer Plasmaspiegel, d.h., eine gleichmässige Verteilung einer ausreichenden Wirkstoffmenge im Plasma, erreicht werden soll, weil das injizierte Material von der Gewebsflüssigkeit beeinflusst wird und damit beispielsweise die physikalisch-chemischen Eigenschaften besagten Materials so verändert werden können, dass ein erheblicher Anteil des injizierten Materials sehr rasch kristallisiert und dadurch an der Injektionsstelle oder in sehr naher Umgebung davon verbleibt. So lassen sich Benzoylharnstoffe, beispielsweise N-[3-(3-Chlor-5-trifluormethylpyridyl-2-oxy)-4-chlorphenyl]-N'-[2,6-difluorbenzoyl]-hamstoff, in Dimethylsulfoxid und N-Methylpyrrolidon (etwa 35 %, Prozentangaben bedeuten hier und im folgenden Gewichtpro-Volumen-Prozent) lösen. Obwohl diese Lösungen mit Wasser mischbar sind, weisen sie den Nachteil auf, dass bei parenteraler Injektion eine erhebliche Menge des injizierten Materials beim Kontakt mit der Gewebsflüssigkeit ausfällt, am Ort der Injektion verbleibt und somit zur Erlangung eines möglichst hohen Plasmaspiegels nichts beiträgt. Der Einsatz solcher Lösungen ist also unökonomisch und insbesondere bei Tieren, welche später geschlachtet und der Ernährung von Tieren oder insbesondere von Menschen dienen sollen, wegen der lokalen Anreicherung an injiziertem Material nur bedingt möglich.

Es wurde nun gefunden, dass sich überraschenderweise durch Zusatz von 1-substituierten Azacycloalkan-2-onen parenteral injizierbare Formulierungen schwerlöslicher Benzoylphenylharnstoffe herstellen lassen, mit denen eine drastisch erhöhte Bioverfügbarkeit erreicht wird, wodurch sich die an sich bekannte hohe Aktivität dieser Wirkstoffklasse auch in dieser exakt dosierbaren Applikationsform ausnutzen lässt. Die erfindungsgemässen Mittel unterscheiden sich von herkömmlichen Mitteln in vorteilhafter Weise dadurch, dass das injizierte Material besser im Plasma verteilt wird und daher wesentlich weniger Material injiziert werden muss, um eine Wirkung gleicher Intensität wie bei herkömmlichen Mitteln zu erreichen, oder dass bei Einsatz gleicher Wirkstoffmengen mit den erfindungsgemässen Mitteln ein hoher Plasmaspiegel über einen deutlich längeren Zeitraum aufrechterhalten werden kann als mit herkömmlichen Mitteln.

Die vorliegende Erfindung betrifft daher ein Mittel zur Bekämpfung von Parasiten, welches parenteral injizierbar ist und dadurch charakterisiert ist, dass es
(a) 0,1 bis 10 %, bevorzugt 0,5 bis 7 und insbesondere 1 bis 5 % mindestens eines Benzoylphenylharnstoffes als Wirkstoff,
(b) 0,1 bis 60 %, bevorzugt 0,1 bis 50 %, insbesondere 0,5 bis 10 % eines 1-substituierten Azacycloalkan-2-ons,
(c) 2 bis 90 %, bevorzugt 40 bis 85 %, eines physiologisch verträglichen Tensids oder Tensidgemisches,
(d) gegebenenfalls als stabilisierende Komponente 0,05 bis 1 % einer Säure oder eines Puffergemisches sowie
(e) ad 100 % eines physiologisch verträglichen hydrophilen Lösungsmittels oder Lösungsmittelgemisches oder ein Gemisch von physiologisch verträglichen hydrophilen und lipophilen Lösungsmitteln enthält.

Diese Angaben sind dahingehend zu verstehen, dass die Komponente e) stets vorhanden ist, d.h., dass die Summe der Komponenten a), b), c) und d) nicht 100 % betragen kann.

Zweckmässigerweise werden Art und Menge des Lösungsmittels zunächst der Art und Menge des Wirkstoffs entsprechend gewählt und später wird, sofern erforderlich, weiteres Lösungsmittel dem Gesamtgemisch ad 100 % zugesetzt, wobei es sich bei dem zum Auffüllen verwendeten Lösungsmittel um das zur Lösung des Wirkstoffs verwendete oder um ein anderes Lösungsmittel handeln kann. Die Bezeichnung "Lösungsmittel" steht hier auch für "Lösungsmittelgemisch".

Unter parenteral injizierbar ist im Rahmen dieser Erfindung zu verstehen, dass das Mittel mit dem gelösten Wirkstoff unter Umgehung des Magen-Darm-Kanals subkutan, intramuskulär oder intravenös mit Hilfe einer Kanüle (Injektionsspritze, Infusion, etc.) in den Körper beispielsweise unter die Haut, in Muskelgewebe oder in Blutgefässe eingebracht werden kann.

Bevorzugt im Rahmen der vorliegenden Erfindung ist die intramuskuläre und vor allem die subkutane Applikation. Erfindungsgemäss lassen sich alle schwerlöslichen Benzoylphenylharnstoffe formulieren und applizieren.

Herstellung und Wirkungweise von Benzoylharnstoffen sind bereits in zahlreichen Publikationen beschrieben, beispielsweise in der PCT-Patentanmeldung WO 86/03941 und den europäischen Patentanmeldungen EP-0,079,311 und EP-0,179,022.

Eine im Rahmen vorliegender Erfindung bevorzugte Gruppe von Benzoylphenylharnstoffen besteht aus Verbindungen der Formel I 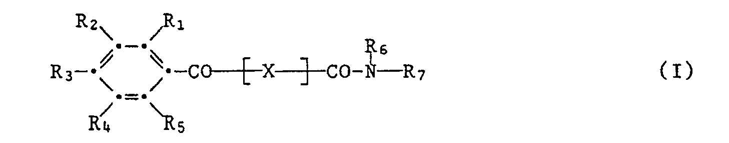 worin
R₁, R₂, R₃ und Rs unabhängig voneinander für Wasserstoff, Halogen, C₁-C₆-Alkyl, C₁-C₆-Haloalkyl, C₁-C₆-Alkoxy, C₁-C₆-Haloalkoxy oder C₁-C₆-Alkylthio stehen;
R₄ für Wasserstoff, Halogen, C₁-C₆-Alkyl, C₁-C₆-Haloalkyl, C₁-C₆-Alkoxy, C₁-C₆-Haloalkoxy; C₁-C₆-Alkylthio oder NHR' steht; worin R' Wasserstoff, R₈CO- oder R₉NHCO- bedeutet, wobei R₈ für ein C₁-C₄-Alkyl steht, welches unsubstituiert oder mit einem bis drei gleichen oder verschiedenen Substituenten aus der Gruppe Halogen, Cᵢ-C₄-Alkoxy, C₁-C₄-Acyloxy und -COOG substituiert ist, worin G Wasserstoff, ein Alkali- oder Erdalkalimetallkation bedeutet, und Rg für eine unsubstituierte oder ein- bis dreifach durch Halogen substituierte C₁-C₄-Alkyl- oder Phenylgruppe steht; 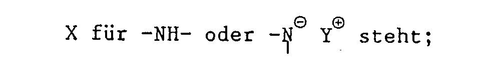 worin
Y^{⊕} ein anorganisches oder organisches Kation repräsentiert;
R₆ Wasserstoff oder C₁-C₆-Alkyl bedeutet; und
R₇ für ein unsubstituiertes oder substituiertes Phenyl, Naphthyl, Pyridyl, Pyridazinyl, Pyrimidinyl oder Pyrazinyl steht, wobei die Substituenten ausgewählt sind aus der Reihe Halogen, insbesondere Fluor oder Chlor; Alkyl, Halogenalkyl, Alkoxy, Halogenalkoxy, Alkylthio, Halogenalkylthio, Alkenyl, Halogenalkenyl, Alkinyl, Halogenalkinyl, Alkylsulfinyl, Alkylsulfonyl, Halogenalkylsulfinyl, Halogenalkylsulfonyl, Alkylamino, Dialkylamino und Benzyl sowie durch Substituenten der Gruppe bestehend aus Halogen, Halogenalkyl, Halogenalkoxy und Nitro substituiertes Phenoxy oder Pyridyloxy, wobei, wenn R₇ für substituiertes Phenyl steht, als Substituent auch Cyan, N'-n-Propyl-N'-phenylureido, eine Brücke -O-CF₂-CF₂-O-, welche zwei benachbarte Kohlenstoffatome des Phenylrings miteinander verbindet, oder Phenoxy, welches durch eine Brücke -O-CF₂-CF₂-0- substituiert ist, welche zwei benachbarte Kohlenstoffatome des Phenylrings miteinander verbindet, vorliegen können (ein Phenylring, welcher durch eine Brücke -O-CF₂-CF₂-O- substituiert ist, welche zwei benachbarte Ringkohlenstoffatome miteinander verbindet, bildet mit besagter Brücke einen 2,2,3,3-Tetrafluor-1,4-benzodioxanrest).

Alkyl als Substituent oder als Bestandteil eines Substituenten steht, sofern die Zahl der Kohlenstoffatome nicht definiert ist, vorzugsweise für unverzweigtes oder verzweigtes C₁-C₆-Alkyl, insbesondere für C,-C₄-Alkyl und bevorzugt für Methyl.

Alkenyl und Alkinyl als Substituenten oder Bestandteile von Substituenten weisen vorzugsweise 3 bis 5 Kohlenstoffatome auf, wobei die Mehrfachbindung im allgemeinen durch mindestens ein an der Mehrfachbindung nicht beteiligtes Kohlenstoffatom vom restlichen Molekül getrennt ist.

Unter Halogen ist Fluor, Chlor, Brom oder Jod, insbesondere aber Fluor oder Chlor, zu verstehen.

Im Rahmen von R₇ sind als Substituenten am Ring oder Ringsystem insbesondere Alkyl-, Halogenalkyl- , Alkylthio-, Alkoxy- und Halogenalkoxygruppen zu verstehen, welche geradkettig oder verzweigt sein können und vorzugsweise 1 bis 4 Kohlenstoffatome aufweisen. Beispiele solcher Gruppen sind u.a. Methyl, -CF₃, Methoxy, Methylthio, -OCF₃, Ethyl, Ethoxy, n-Propyl, -CF₂-CHF-CF₃, n-Propoxy, -OCF₂-CHF-CF₃, Isopropyl, Isopropoxy, n-Butyl, n-Butoxy, n-Pentyl, n-Pentyloxy, n-Hexyl, n-Hexyloxy.

Hervorzuheben sind Verbindungen der Formel I, worin
R₁, R₂, R₃ und R₅ unabhängig voneinander für Wasserstoff, Halogen, C₁-C₆-Alkyl, Ci-C₆-Haloalkyl, C₁-C₆-Alkoxy, C₁-C₆-Haloalkoxy oder C₁-C₆-Alkylthio stehen;
R₄ für Wasserstoff, Halogen, Ci-C₆-Alkyl, C₁-C₆-Haloalkyl, C₁-C₆-Alkoxy, C₁-C₆-Haloalkoxy; C₁-C₆-Alkylthio oder NHR' steht; worin R' Wasserstoff, R₈CO- oder R₉NHCO- bedeutet, wobei R₈ für ein C₁-C₄-Alkyl steht, welches unsubstituiert oder mit einem bis drei gleichen oder verschiedenen Substituenten aus der Gruppe Halogen, C₁-C₄-Alkoxy, C₁-C₄-Acyloxy und -COOG substituiert ist, worin G Wasserstoff, ein Alkali- oder Erdalkalimetallkation bedeutet, und Rg für eine unsubstituierte oder ein- bis dreifach durch Halogen substituierte C₁-C₄-Alkyl- oder Phenylgruppe steht; 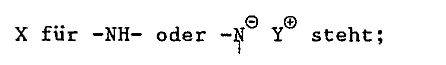 worin
Y^{⊕} ein anorganisches oder organisches Kation repräsentiert;
R₆ Wasserstoff oder Ci-C₆-Alkyl bedeutet; und
R₇ für ein unsubstituiertes oder substituiertes Phenyl, Naphthyl, Pyridyl, Pyridazinyl, Pyrimidinyl oder Pyrazinyl steht, wobei die Substituenten ausgewählt sind aus der Reihe Halogen, insbesondere Fluor oder Chlor; Alkyl, Halogenalkyl, Alkoxy, Halogenalkoxy, Alkylthio, Halogenalkylthio, Alkenyl, Halogenalkenyl, Alkinyl, Halogenalkinyl, Alkylsulfinyl, Alkylsulfonyl, Halogenalkylsulfinyl, Halogenalkylsulfonyl, Alkylamino, Dialkylamino und Benzyl sowie durch Halogen, Halogenalkyl, Halogenalkoxy oder Nitro substituiertes Phenoxy oder Pyridyloxy.

Besonders bevorzugt sind Vertreter der Formel I worin
R₁ und R₅ unabhängig voneinander für Wasserstoff, Fluor, Chlor, Methoxy oder Methylthio, insbesondere Fluor stehen;
R₃ Wasserstoff oder Fluor und R4 Wasserstoff oder NH₂ repräsentieren;
R₂ für Wasserstoff, Fluor oder Chlor, insbesondere Wasserstoff steht; 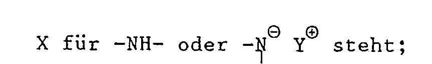 worin
Y^{⊕} für Na^{⊕}, K^{⊕} oder Tetraalkylammonium, wie (n-C₄H₉)₄N^{⊕}, (CH₃)₄N^{⊕}, (C₂H₅)₄N^{⊕} oder n-C₁₆H₃₃-N^{⊕}(CH₃)₃, insbesondere jedoch für -NH- steht;
R₆ für Wasserstoff oder C₁-C₃-Alkyl, vorzugsweise Wasserstoff steht und
R₇ für unsubstituiertes oder vorzugsweise substituiertes Phenyl steht, wobei der Phenylrest vorzugsweise durch ein bis zwei Halogenatome, insbesondere Fluor oder Chlor und zusätzlich entweder durch Ci-C₆-Halogenalkoxy, insbesondere C₁-C₃-Haloalkoxy, oder durch 2-Pyridyloxy substituiert ist, wobei der 2-Pyridyloxyrest seinerseits vorzugsweise durch CF₃ und Halogen, insbesondere CF₃ und Fluor oder Chlor substituiert ist.

Eine weitere Gruppe bevorzugter Benzoylphenylharnstoffe im Rahmen der Formel I besteht aus folgenden Verbindungen der Formel II 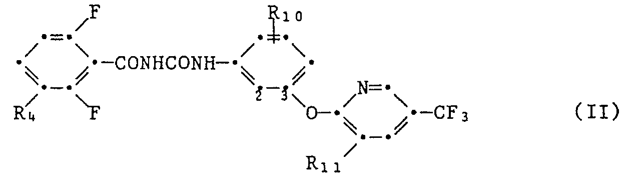 worin
R₄ Wasserstoff oder NH₂ bedeutet;
R₁₀ für Wasserstoff, Halogen oder Methyl steht; und
R₁₁ Wasserstoff oder Halogen bedeutet.

Eine bevorzugte Untergruppe bilden Verbindungen der Formel 11, worin
R₄ Wasserstoff oder NH₂ bedeutet;
R₁₀ für Wasserstoff, Fluor, Chlor oder Brom steht; und
R₁₁ Wasserstoff, Fluor, Chlor oder Brom bedeutet.

Besonders bevorzugte Vertreter der Formel II sind solche, worin
R₄ Wasserstoff oder NH₂ bedeutet;
R₁₀ für 4-Fluor, 4-Chlor, 4-Brom oder 4-Methyl steht; und
R₁₁ Chlor bedeutet.

Als besonders bevorzugte Einzelvertreter von Verbindungen der Formel II sind die der nachfolgenden Tabelle genannt:

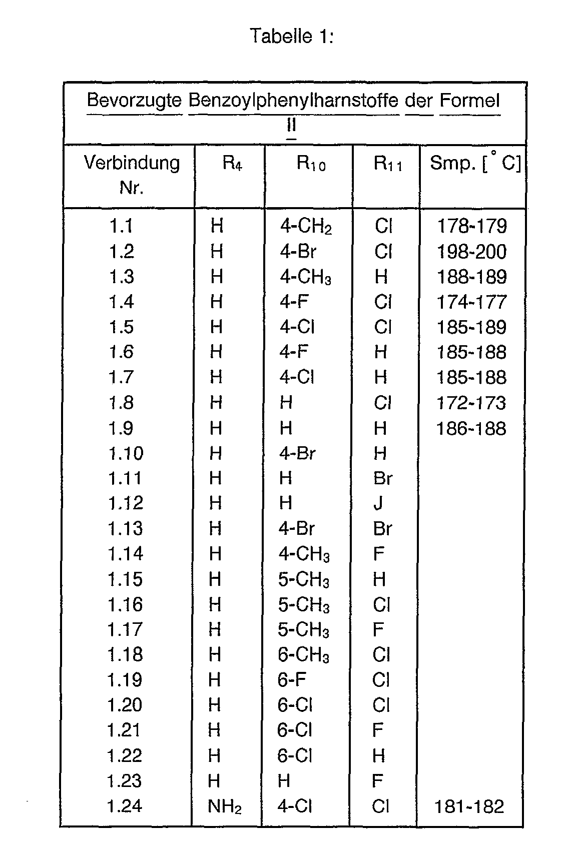

Bevorzugte Ausführungsformen der vorliegenden Erfindung enthalten als Wirkstoff einen der nachfolgend aufgelisteten aber nicht abschliessend genannten Benzoylphenylharnstoffe:

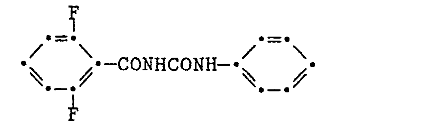

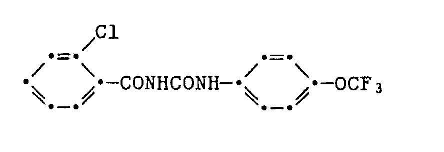

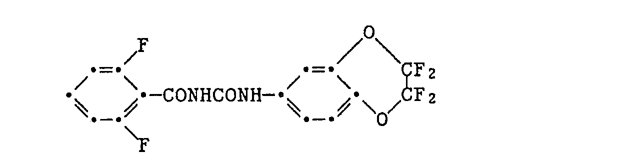

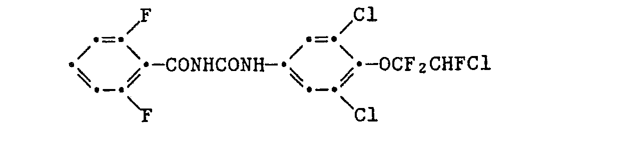

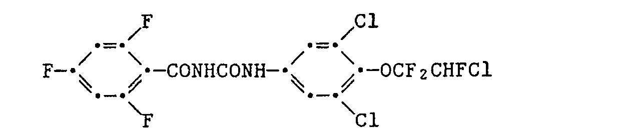

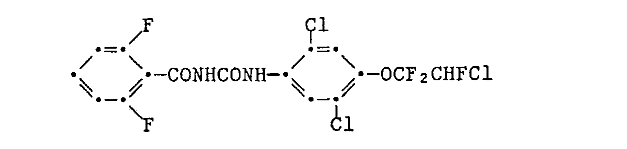

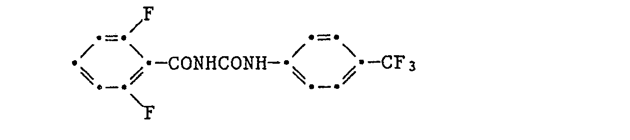

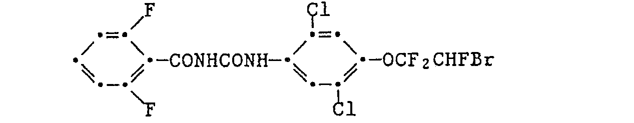

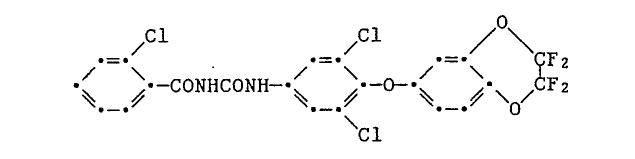

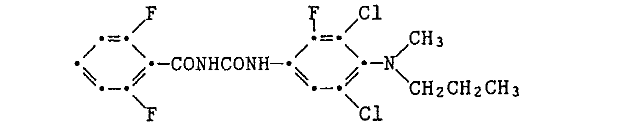

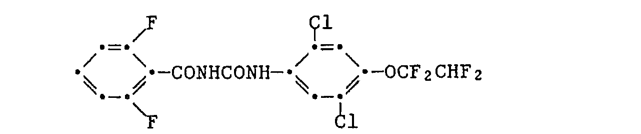

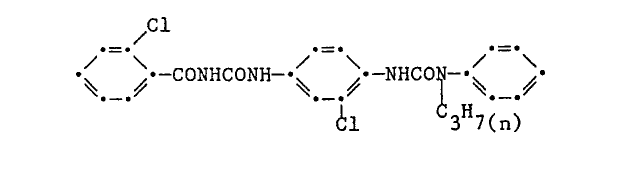

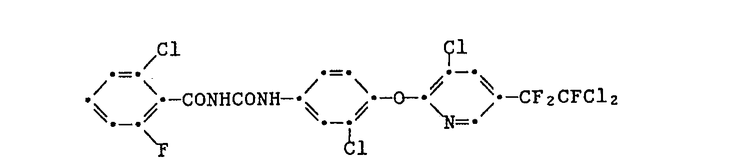

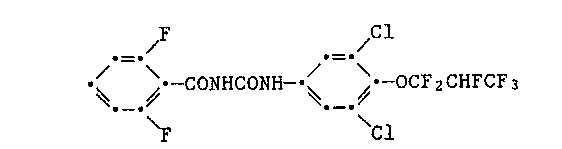

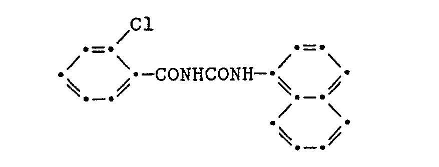

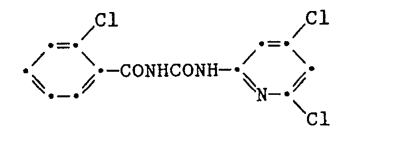

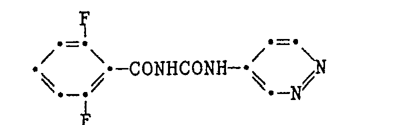

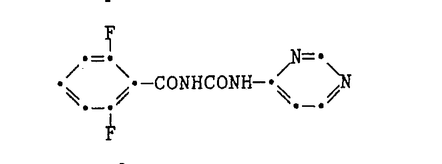

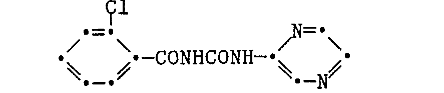

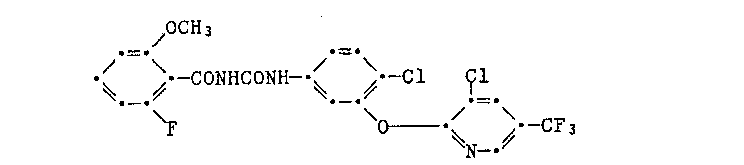

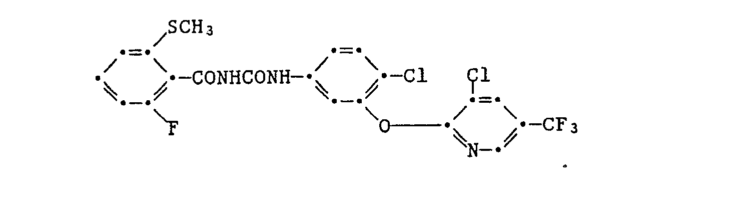

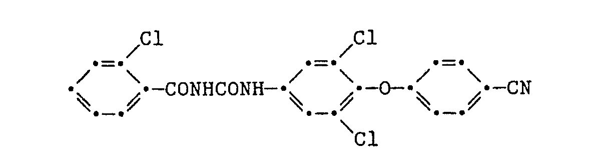

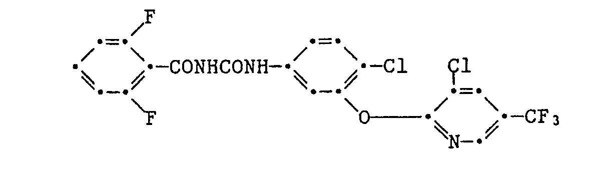

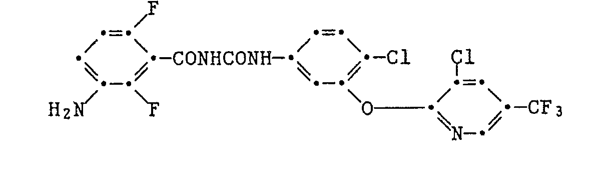

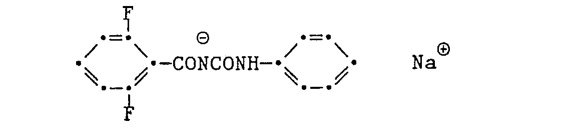

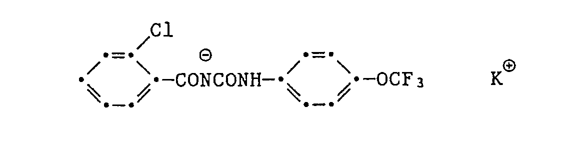

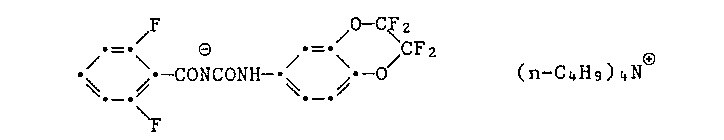

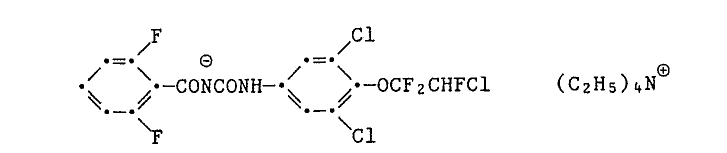

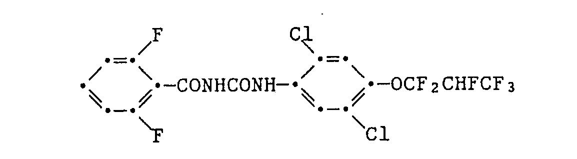

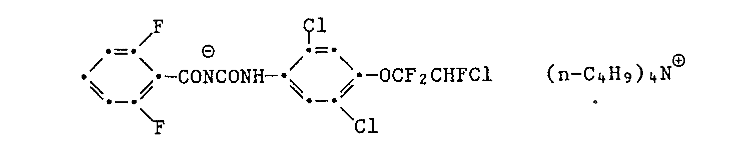

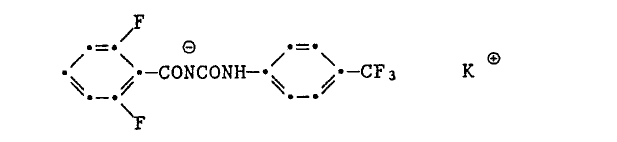

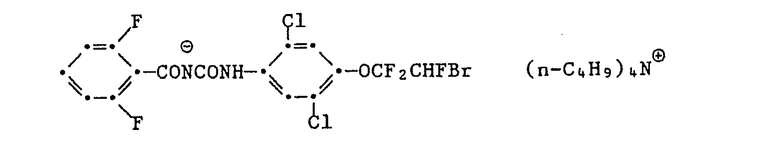

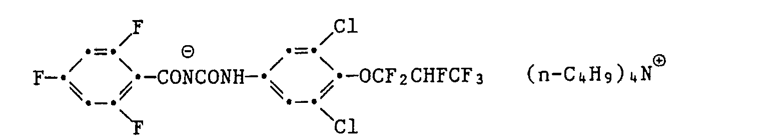

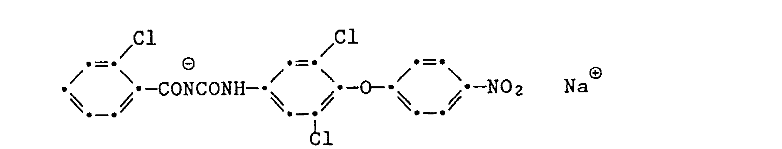

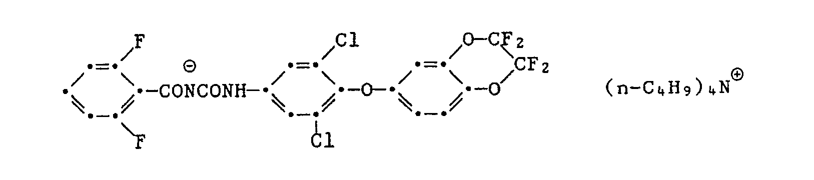

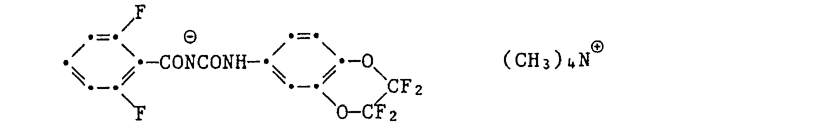

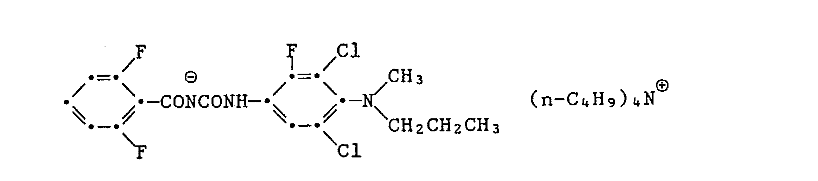

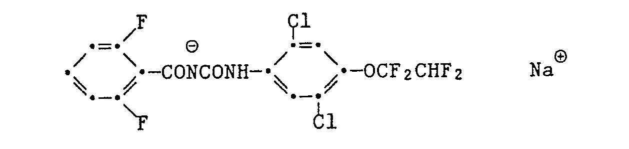

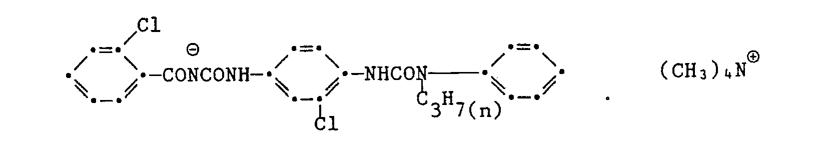

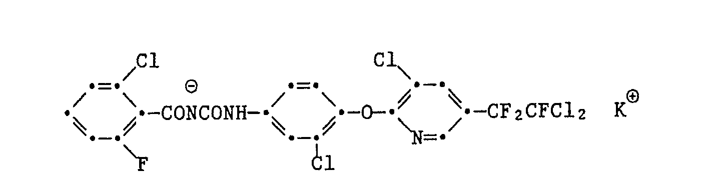

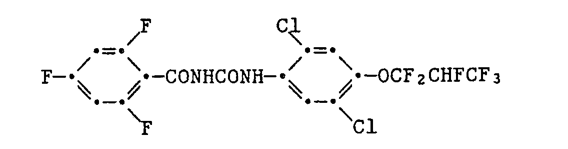

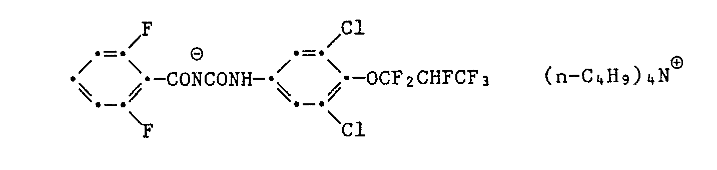

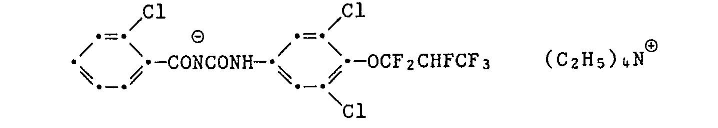

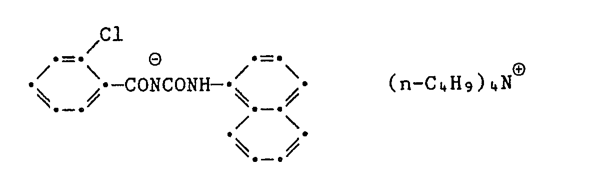

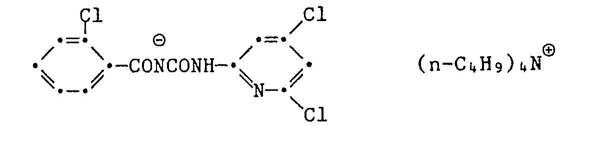

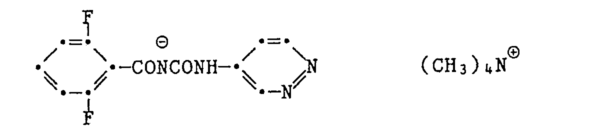

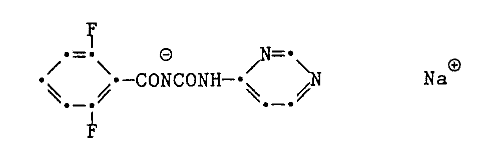

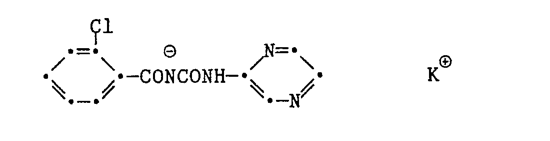

Besonders bevorzugt sind erfindungsgemässe Formulierungen, die N-[3-(3-Chlor-5-trifluormethylpyridyl-2-oxy)-4-chlorphenyl]-N'-[2,6-difluorbenzoyl]-harnstoff oder N-[3-(3-Chlor-5-trifluormethylpyridyl-2-oxy)-4- chlorphenyl]-N'-[2,6-difluor-3-aminobenzoyl]-harnstoff als Wirkstoff enthalten.

Im Rahmen vorliegender Erfindung sind unter 1-substituierten Azacycloalkan-2-onen Verbindungen der Formel III zu verstehen 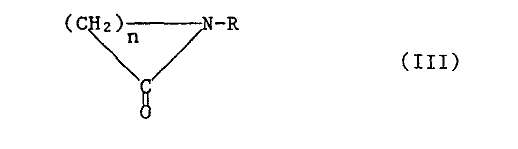 wobei n eine ganze Zahl von 2 bis 7 bedeutet und R für ein C₆-C₁₅-Alkyl steht, das gegebenenfalls durch ein Sauerstoffatom unterbrochen sein kann. R ist vorzugsweise ein unverzweigtes C₁₀-C₁₄-Alkyl, insbesondere n-Dodecyl. Besonders bevorzugt unter den 1-substituierten Azacycloalkan-2-onen sind 1-n-Dodecylazacycloheptan-2-on (Azoneo) und 1-n-Dodecylazacyclopentan-2-on.

Die Verwendung von 1-substituierten Azacycloalkan-2-onen einschliesslich Azon (Azone®) als Penetrationsverstärker in transdermalen Systemen - Eindringen in und durch die Haut nach topikaler Applikation - ist beispielsweise aus US 4 557 934 und der korrespondierenden Patentpublikation EP 0 129 284 bekannt. Beschrieben wird die Schlepperfunktion von 1-substituierten Azacycloalkan-2-onen, was einem verbesserten Durchdringen der Hautbarriere für bestimmte Wirkstoffe gleichkommt. In den japanischen Patentanmeldungen JP 63-002 923 und JP 61-263 914 wird Azon als Formulierungshilfsstoff in Präparaten zur Bekämpfung von Tumoren offenbart.

Im Rahmen der vorliegenden Erfindung kommen als physiologisch verträgliche Tenside vor allem nichtionische Tenside in Betracht.

Im Rahmen der vorliegenden Erfindung kommen als physiologisch verträgliche Tenside vor allem nichtionische Tenside mit einem Molekulargewicht unterhalb 20 000, vorzugsweise unterhalb 5 000 in Betracht, welche zur nachstehend definierten Gruppe gehören:
(1) Polyethoxylierte Triglyceride, (2) polyethoxylierte Hydroxystearinsäureester, (3) polyethoxylierte Sorbitan-Fettsäureester, (4) Sorbitan-Fettsäureester, (5) polyethoxylierte Methylglukosid-Sesquistearate, (6) Fettsäurezuckerester, (7) polyethoxylierte Fettalkoholether, (8) polyethoxylierte Fettsäureester, (9) polyethoxylierte Fettsäureamine und (10) Polyethoxy-polypropoxy-Blockpolymerisate [bevorzugt Blockpolymerisate mit HLB-Werten (HLB: hydrophile-lipophile-balance) von 12-20].
Von den polyethoxylierten Triglyceriden, Hydroxystearinsäureestern, Sorbitan-Fettsäureestern, Methylglukosid-Sesquistearaten, Fettalkoholethern, Fettsäureestern, und Fettsäureaminen sind vor allem solche mit 2 bis 100, insbesondere 10 bis 40 und vor allem mit 10, 20 oder 40 Ethylenoxideinheiten zu erwähnen.

Als Polyethoxy-polypropoxy-Blockpolymerisate sind diejenigen mit einem Ethylenoxidanteil von 20 bis 80 % hervorzuheben.

Besonders geeignete Vertreter aus der vorstehend angeführten Gruppe sind (EO = Zahl der Ethylenoxideinheiten):
(1) Polyethoxyliertes Rizinusöl (EO 40), im Handel erhältlich unter der Bezeichnung CREMOPHOR@ EL (BASF AG);
Polyethoxyliertes, hydriertes Rizinusöl (EO 40, EO 60), im Handel erhältlich unter der Bezeichnung CREMOPHOR@ RH 40, RH60, (BASF AG);
(2) Polyethoxylierter 12-Hydroxystearinsäureester (EO 15), im Handel erhältlich unter der Bezeichnung SOLUTOL@ HS 15 (BASF);
(3) Polyethoxyliertes Sorbitanmonolaurat (EO 20), im Handel erhältlich unter der Bezeichnung TWEEN@ 20 (ICI);
Polyethoxyliertes Sorbitanmonopalmitat (EO 20), im Handel erhältlich unter der Bezeichnung TWEEN@ 40 (ICI);
Polyethoxyliertes Sorbitanmonostearat (EO 20), im Handel erhältlich unter der Bezeichnung TWEEN@ 60 (ICI);
Polyethoxyliertes Sorbitanmonooleat (EO 20), im Handel erhältlich unter der Bezeichnung TWEEN@ 80 (ICI);
Polyethoxyliertes Sorbitantristearat (EO 20), im Handel erhältlich unter der Bezeichnung TWEEN@ 65 (ICI);
Polyethoxyliertes Sorbitantrioleat (EO 20), im Handel erhältlich unter der Bezeichnung TWEEN@ 85 (ICI);
(4) Sorbitanmonolaurat, im Handel erhältlich unter der Bezeichnung SPAN® 20 (ICI);
Sorbitanmonopalmitat, im Handel erhältlich unter der Bezeichnung SPAN@ 40 (ICI);
Sorbitanmonostearat, im Handel erhältlich unter der Bezeichnung SPAN@ 60 (ICI);
Sorbitanmonooleat, im Handel erhältlich unter der Bezeichnung SPAN@ 80 (ICI);
Sorbitantristearat, im Handel erhältlich unter der Bezeichnung SPAN@ 65 (ICI);
Sorbitantrioleat, im Handel erhältlich unter der Bezeichnung SPAN@ 85 (ICI);
(5) Polyethoxyliertes Methylglukosid-sesquistearat (EO 20), im Handel erhältlich unter der Bezeichnung GLUCAMATE@ SSE-20 (Amerchol Corp.);
(6) 12-Hydroxystearinsaccharoseester,
Saccharosemonolaurat,
Saccharosemonomyristat,
Saccharosemonopalmitat,
Saccharosemonooleat,
Saccharosemonostearat,
Saccharosedistearat,
Saccharosedioleat,
Saccharosedipalmitat,
Saccharose-mono-/di-/tri-palmitat/stearat-Produkte sind im Handel erhältlich, beispielsweise unter den Bezeichnungen CRODESTA@ DKS F10, F20, F50, F70, F110, F140, F160 (Croda Chemicals Ltd.);
Saccharosemonococoat, im Handel erhältlich unter der Bezeichnung CRODESTAO SL40 (Croda Chemicals Ltd.);
(7) Polyethoxylierter Oleylalkoholether (EO 2), im Handel erhältlich unter den Bezeichnungen AMEROXOL® OE-2 (Amerchol Europe) und Brij 92 (Atlas Chemie/ICI);
Polyethoxylierter Oleylalkoholether (EO 10), im Handel erhältlich unter den Bezeichnungen AMEROXOL® OE-10 (Amerchol Europe) und Brij 96 (Atlas Chemie/ICI);
Polyethoxylierter Oleylalkoholether (EO 20), im Handel erhältlich unter den Bezeichnungen AMEROXOL® OE-20 (Amerchol Europe) und Brij 98 (Atlas Chemie/ICl);
(8) Polethoxyliertes Stearat (EO 8, EO 20, EO 30, EO 40^{*}, EO 50, EO 100), im Handel erhältlich unter den Bezeichnungen Myrj@ 45, 49, 51, (52, 52C, 52S)^{*}, 53, 59 (ICI);
Polyethoxyliertes Laurat (als Dilaurat) (EO 5, EO 10), im Handel erhältlich unter der Bezeichnung Pegosperse® 200-DL (Glyco Inc.);
Polyethoxyliertes Cocoat (EO 7), im Handel erhältlich unter der Bezeichnung CETIOL® HE (Henkel Corp.);
(9) Polyethoxyliertes Talgfettamin (EO 10), im Handel erhältlich unter der Bezeichnung GENAMIN® T100 (Hoechst AG);
(10) Ethylenoxid-Propylenoxid-Blockpolymerisat mit einem Molekulargewicht von etwa 16000 und einem Ethylenoxidanteil von 80 %, im Handel erhältlich unter der Bezeichnung PLURONIC® F-108 (BASF Wyandotte Corp.);
Ethylenoxid-Propylenoxid-Blockpolymerisat mit einem Molekulargewicht von etwa 4500 und einem Ethylenoxidanteil von 50 %, im Handel erhältlich unter der Bezeichnung PLURONIC® P-85 (BASF Wyandotte Corp.);
Ethylenoxid-Propylenoxid-Blockpolymerisat mit einem Molekulargewicht von etwa 1450 und einem Ethylenoxidanteil von 20 %, im Handel erhältlich unter der Bezeichnung PLURONIC@ L-42 (BASF Wyandotte Corp.);
Ethylenoxid-Propylenoxid-Blockpolymerisat mit einem Molekulargewicht von etwa 2900 und einem Ethylenoxidanteil von 40 %, im Handel erhältlich unter den Bezeichnungen SYNPERONICO PE L 64 (ICI) und Pluronic L 64 (BASF Wyandotte Corp.).

Die vorgängig angeführten Handelsbezeichnungen für Tenside sind nur als Beispiele, nicht als Beschränkungen zu verstehen.

Im Rahmen der vorliegende Erfindung erweisen sich folgende Tenside (A bis F) und Tensidmischungen (X bis Z) als besonders geeignet:
A) Polyethoxyliertes Rizinusöl (EO 40);
B) Polyethoxyliertes hydriertes Rizinusöl (EO 40);
C) Polyethoxylierter 12-Hydroxystearinsäureester (EO 15);
D) Polyethoxylierter Oleylalkoholether (EO 10);
E) Polyethoxyliertes Laurat (als Dilaurat; Molekulargewicht 200, 400, EO 5, EO 10);
F) Ethylenoxid-Propylenoxid-Blockpolymerisat (Molekulargewicht 2900, Ethylenoxidanteil 40 %;
X) Mischung von A) und B), vorzugsweise im Verhältnis A:B = 2:1 bis 1:2, insbesondere 1:1;
Y) Mischung von C) und E), vorzugsweise im Verhältnis von C:E = 2:1 bis 1:2, insbesondere 1:1;
Z) Mischung von A) und E), vorzugsweise im Verhältnis von A:E = 3:1 bis 1:2, insbesondere 1:1 bis 1,5:1.

Nichtionische Tenside, wie sie sich im Rahmen der vorliegenden Erfindung verwenden lassen, sind aus Standardwerken der einschlägigen Literatur bekannt. Als Beispiele für solche Standardwerke seien genannt: Ash, M. und 1., Encyclopedia of Surfactants, Chemical Publishing Co. Inc., New York, N.Y. (Vol. I, 1980; Vol. 11, 1981; Vol. 111, 1981; Vol IV, 1985);
1986 International McCutcheon's Emulsifiers & Detergents, The Manufacturing Confectioner Publishing Co., Glen Rock, NJ, USA;
Stache, H., Tensid-Taschenbuch, Carl Hanser Verlag, München, Wien, 1981.

Als physiologisch verträgliche hydrophile Lösungsmittel kommen im Rahmen der vorliegenden Erfindung solche in Betracht, welche zur nachstehend aufgeführten Gruppe gehören: a) Monohydroxyalkylgruppen mit 2 bis 10 Kohlenstoffatomen, b) acyclische gesättigte Polyole, c) Dimethylsulfoxid, d) Glycerinformal, e) 2,2-Dimethyl-4-hydroxymethyl-1,3-dioxolan (Solketal), f) Tetrahydrofurfurylalkohol-(polyethoxy)-ether (Glykofurol), g) Wasser und h) N-Methylpyrrolidon.

Als Beispiele für Vertreter der Untergruppe a) seien Ethanol, 1-Propanol, 2-Propanol, 2-Butanol, tert.-Butylalkohol, 1-Hexanol, 1-Heptanol, 2-Heptanol, 1-Octanol, 2-Octanol, 1-Nonanol und 1-Decanol genannt.

Beispiele für Vertreter der Untergruppe b) sind vor allem Polyole mit 2 bis 6, vorzugsweise 3 bis 4, Kohlenstoffatomen und 2 bis 3 Hydroxygruppen, wie 1,2-Propandiol, Glycerin, 1,2-Butandiol, 1,3-Butandiol sowie entsprechende, mit niederen Alkylgruppen, insbesondere Methylgruppen, veretherte Polyole wie beispielsweise 1,2-Propandiol-1-methylether, und des weiteren Polyethylenglykole mit einer mittleren Molmasse im Bereich von 200 bis 600, wie beispielsweise Polyethylenglykol 300 (im Handel erhältlich beispielsweise unter der Bezeichnung PLURIOL E300, BASF).

Als lipophile Lösungsmittel kommen vor allem Ester von Carbonsäuren, beispielsweise Ethylacetat, n-Propylacetat und n-Butylacetat sowie flüssige Wachse in Betracht, wie beispielsweise Isopropylmyristat, Isopropylpalmitat, Laurinsäurehexylester und Ethyloleat.

Bei Mischungen von hydrophilen mit lipophilen Lösungsmitteln beträgt der Anteil an lipophilem Lösungsmittel vorteilhafterweise 0,1 bis 30 %, bezogen auf die gesamte Lösungsmittelmenge.

Im Rahmen der vorliegenden Erfindung sind alle Tenside und Lösungsmittel einsetzbar, die normalerweise für veterinärmedizinische Verabreichungen in Frage kommen.

Als stabilisierende Komponente im Rahmen dieser Erfindung kommen als Säuren insbesondere organische Säuren mit 3 bis 6 Kohlenstoffatomen in Betracht. Beispiele für geeignete Säuren sind Zitronensäure, Ascorbinsäure, Milchsäure, Aepfelsäure und Weinsäure.

Als stabilisierende Puffergemische eignen sich physiologisch verträgliche, in der Humanmedizin oder in der Veterinärmedizin üblicherweise verwendete Puffergemische.

Zu den bevorzugten Ausführungsformen der erfindungsgemässen, injizierbaren parasitiziden Mittel gehören beispielsweise folgende Zusammensetzungen:
(A) 0,1 bis 10 % eines Benzoylphenylharnstoffes, 0,5 bis 10 % eines 1-substituierten Azacycloalkan-2- ons, 2 bis 90 % eines Tensids oder Tensidgemisches, 0,05 bis 1 % eines Stabilisators und ad 100 % Lösungsmittel;
(B) 0,5 bis 7 % eines Benzoylphenylharnstoffes, 0,5 bis 10 % eines 1-substituierten Azacycloalkan-2- ons, 2 bis 90 % eines Tensids oder Tensidgemisches, 0,05 bis 1 % eines Stabilisators und ad 100 % Lösungsmittel;
(C) 1 bis 5 % eines Benzoylphenylharnstoffes, 0,5 bis 10 % eines 1-substituierten Azacycloalkan-2-ons, 2 bis 90 % eines Tensids oder Tensidgemisches, 0,05 bis 1 % eines Stabilisators und ad 100 % Lösungsmittel;
(D) 0,1 bis 10 % eines Benzoylphenylharnstoffes, 0,1 bis 50 % eines 1-substituierten Azacycloalkan-2- ons, 2 bis 90 % eines Tensids oder Tensidgemisches, 0,05 bis 1 % eines Stabilisators und ad 100 % Lösungsmittel;
(E) 0,5 bis 7 % eines Benzoylphenylharnstoffes, 0,1 bis 50 % eines 1-substituierten Azacycloalkan-2-ons, 2 bis 90 % eines Tensids oder Tensidgemisches, 0,05 bis 1 % eines Stabilisators und ad 100 % Lösungsmittel;
(F) 1 bis 5 % eines Benzoylphenylharnstoffes, 0,1 bis 50 % eines 1-substituierten Azacycloalkan-2-ons, 2 bis 90 % eines Tensids oder Tensidgemisches, 0,05 bis 1 % eines Stabilisators und ad 100 % Lösungsmittel;
(G) 0,1 bis 10 % eines Benzoylphenylharnstoffes, 0,1 bis 60 % eines 1-substituierten Azacycloalkan-2- ons, 2 bis 90 % eines Tensids oder Tensidgemisches, 0,05 bis 1 % eines Stabilisators und ad 100 % Lösungsmittel;
(H) 0,5 bis 7 % eines Benzoylphenylharnstoffes, 0,1 bis 60 % eines 1-substituierten Azacycloalkan-2- ons, 2 bis 90 % eines Tensids oder Tensidgemisches, 0,05 bis 1 % eines Stabilisators und ad 100 % Lösungsmittel;
(I) 1 bis 5 % eines Benzoylphenylharnstoffes, 0,1 bis 60 eines 1-substituierten Azacycloalkan-2-ons, 2 bis 90 % eines Tensids oder Tensidgemisches, 0,05 bis 1 % eines Stabilisators und ad 100 % Lösungsmittel;

Innerhalb der Kombinationen (A) bis (I) sind als spezifische Ausführungsformen insbesondere solche bevorzugt, worin der Wirkstoff N-[3-(3-Chlor-5-trifluormethylpyridyl-2-oxy)-4-chlorphenyl]-N'-[2,6- difluorbenzoyl]- harnstoff oder N-[3-(3-Chlor-5-trifluormethylpyridyl-2-oxy)-4-chlorphenyl]-N'-[2,6-difluor-3-aminobenzoyl]-harnstoff ist.

Innerhalb der Kombinationen (A) bis (I) sind weiterhin jene besonders bevorzugt, worin das 1- substituierte Azacycloalkan-2-on n-Dodecylazacycloheptan-2-on oder n-Dodecylazacyclopentan-2-on ist.

Die vorliegende Erfindung betrifft ferner ein Verfahren zur Herstellung eines injizierbaren Mittels zur Bekämpfung von Parasiten am Nutz- und Haustier, das parenteral injizierbar ist und 0,1 bis 10 % eines Benzoylphenylharnstoffes als Wirkstoff, 0,1 bis 60 % eines 1-substituierten Azacycloalkan-2-ons, 2 bis 90 % eines physiologisch verträglichen Tensids oder Tensidgemisches und gegebenenfalls als stabilisierende Komponente 0,05 bis 1 % einer Säure oder eines Puffergemisches sowie ad 100 % eines physiologisch verträglichen hydrophilen Lösungsmittels oder Lösungsmittelgemisches oder ein Gemisch von physiologisch verträglichen hydrophilen und lipophilen Lösungsmitteln enthält, dadurch gekennzeichnet, dass der Benzoylphenylharnstoff in einem hydrophilen Lösungsmittel gelöst und mit dem Tensid sowie dem 1- substituierten Azacycloalkan-2-on, gegebenenfalls dem lipophilen Lösungsmittel und gegebenenfalls einem Stabilisator vermischt, mit einem Lösungsmittel aufgefüllt und die so erhaltene Endmischung durch ein Filter sterilfiltriert und anschliessend hitzesterilisiert wird.

Die erfindungsgemässen, injizierbaren Mittel sind gegen eine Vielzahl von Parasiten wirksam, besonders gegen Ektoparasiten an Haus- und Nutztieren, wie Insekten der Ordnungen Homoptera, Heteroptera, Diptera, Thysanoptera, Orthoptera, Anoplura, Siphonaptera, Psocoptera und Hymenoptera sowie Spinnentiere (Arachnida) der Ordnung Acarina, insbesondere der Unterordnung Ixodida (Zecken). Die erfindungsgemässen Mittel zeigen auch eine gute anthelminthische Wirkung. Sie sind zur Bekämpfung parasitärer Nematoden, beispielsweise der Ordnungen Rhabditida, Ascaridida, Spirurida, Trichocephalida, oder zur Bekämpfung von Cestoden der Ordnungen Cyclophyllidae, Pseudophillidae oder zur Bekämpfung von Trematoden der Ordnung Digenea bei Haus- und Nutztieren geeignet.

Unter Haus- und Nutztieren sind beispielsweise Rinder, Schafe, Ziegen, Pferde, Schweine, Katzen und Hunde zu verstehen.

Der besondere Vorteil der erfindungsgemässen Mittel liegt darin, dass man sie systemisch einsetzen kann, indem man sie lokal appliziert und so ihre Ausbreitung über die Blut- und Gewebeflüssigkeit im gesamten Tier bewirkt.

Die vorliegende Erfindung betrifft daher auch ein Verfahren zur systemischen Bekämpfung von Parasiten am Nutz- und Haustier, welches darin besteht, dass man das erfindungsgemässe Mittel prophylaktisch oder kurativ in das Tier parenteral injiziert.

Des weiteren betrifft die vorliegende Erfindung die Verwendung von 1-substituierten Azacycloalkan-2- onen in veterinärmedizinischen Präparaten zur Bekämpfung von Endo- und Ektoparasiten auf der Basis von Benzoylphenylharnstoffen.

### Beispiele

Zusammensetzung erfindungsgemässer Mittel

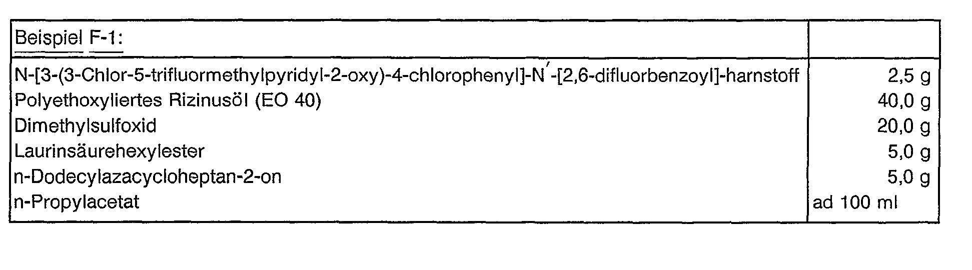

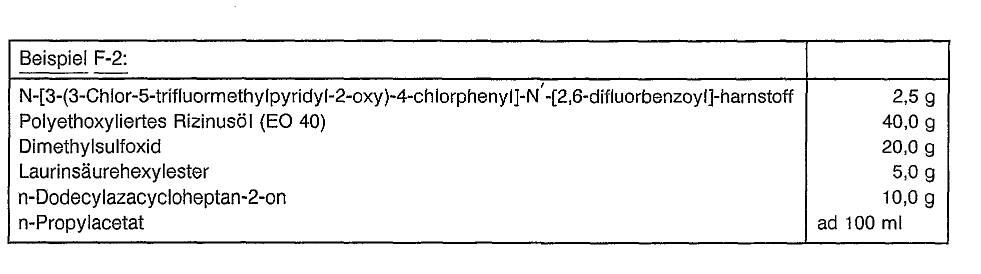

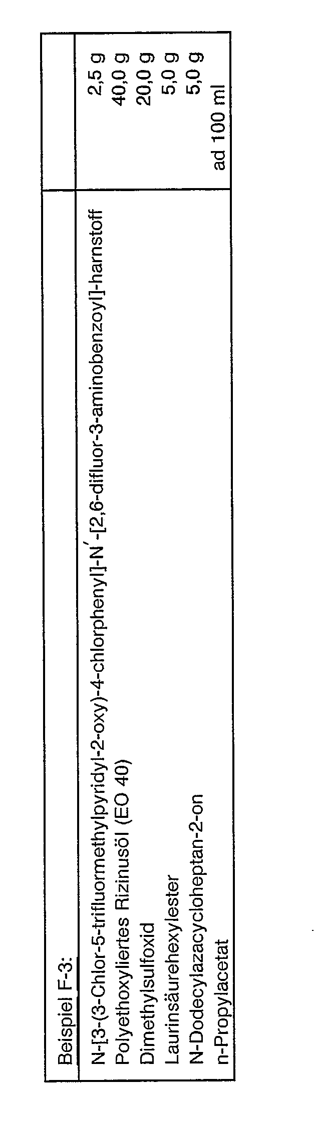

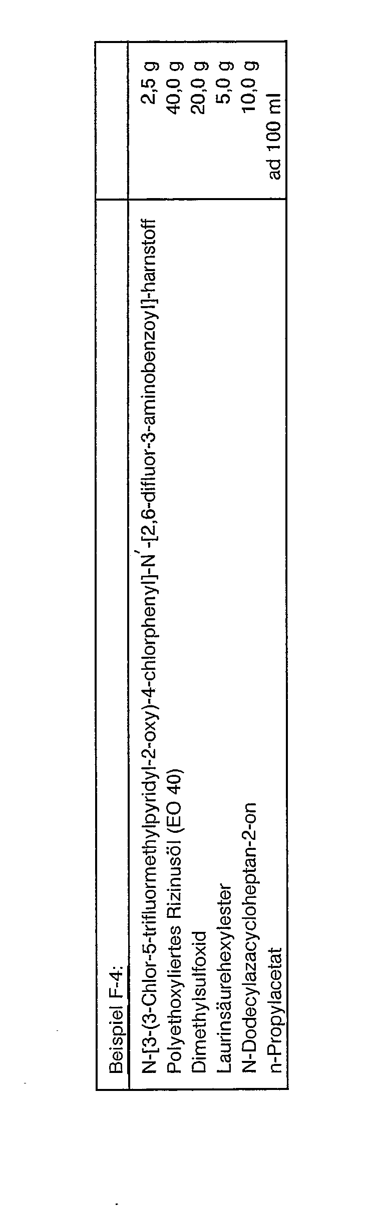

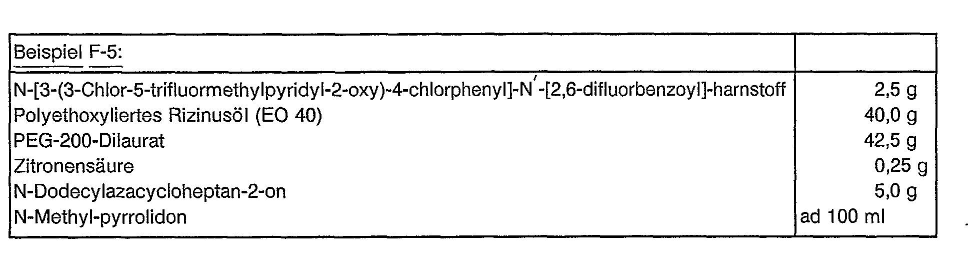

### Biologisches Beispiel

### Bioverfügbarkeit (Plasmakonzentration)

Zur Ueberprüfung der Bioverfügbarkeit in vivo werden die erfindungsgemässen Formulierungen F-1 bis F-5 je 4 Rindern von 200 bis 300 kg Körpergewicht (KG) in einer Dosierung von 1 mg/kg KG einmalig subcutan appliziert. Die aus 4 Rindern bestehende Kontrollgruppe erhält zum Vergleich die Wirksubstanz in folgender Formulierung:

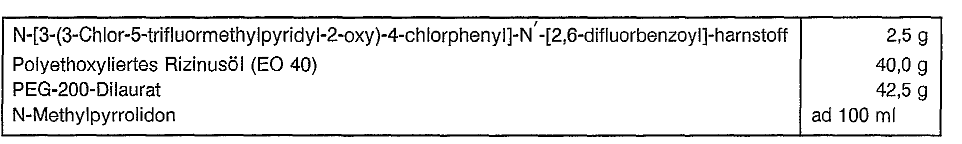

Die nach bestimmten Zeitintervallen entnommenen Plasmaproben werden analysiert. Während die erfindungsgemässen Formulierungen nach 24 Stunden Plasmakonzentrationen zwischen 50 und 100 ppb erreichen, liegt die Konzentration der Aktivsubstanz bei der nicht erfindungsgemässen Zubereitung um mindestens einen Faktor 2 darunter.

## Patentansprüche

1. Mittel zur Bekämpfung von Parasiten, die Nutz- und Haustiere befallen, dadurch gekennzeichnet, dass es parenteral injizierbar ist und 0,1 bis 10 % eines Benzoylphenylharnstoffes als Wirkstoff, 0,1 bis 60 % eines 1-substituierten Azacycloalkan-2-ons, 2 bis 90 % eines physiologisch verträglichen Tensids oder Tensidgemisches und gegebenenfalls als stabilisierende Komponente 0,05 bis 1 % einer Säure oder eines Puffergemisches sowie ad 100 % eines physiologisch verträglichen hydrophilen Lösungsmittels oder Lösungsmittelgemisches oder ein Gemisch von physiologisch verträglichen hydrophilen und lipophilen Lösungsmitteln enthält.

2. Mittel nach Anspruch 1, dadurch gekennzeichnet, dass es Verbindungen der Formel I 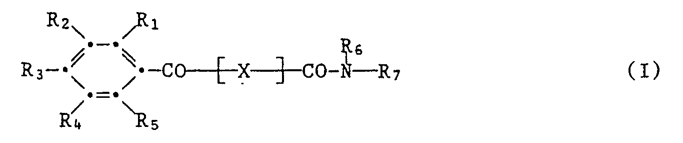 enthält, worin
R₁, R₂, R₃ und R₅ unabhängig voneinander für Wasserstoff, Halogen, Ci-C₆-Alkyl, C₁-C₆-Haloalkyl, Ci-C₆-Alkoxy, C₁-C₆-Haloalkoxy oder C₁-C₆-Alkylthio stehen;
R₄ für Wasserstoff, Halogen, Cₗ-C₆-Alkyl, C₁-C₆-Haloalkyl, Cₗ-C₆-Alkoxy, C₁-C₆-Haloalkoxy; C₁-C₆-Alkylthio oder NHR' steht; worin R Wasserstoff, R₈CO- oder R₉NHCO- bedeutet, wobei R₈ für ein C₁-C₄-Alkyl steht, welches unsubstituiert oder mit einem bis drei gleichen oder verschiedenen Substituenten aus der Gruppe Halogen, C₁-C₄-Alkoxy, C₁-C₄-Acyloxy und -COOG substituiert ist, worin G Wasserstoff, ein Alkali- oder Erdalkalimetallkation bedeutet, und Rg für eine unsubstituierte oder ein- bis dreifach durch Halogen substituierte C₁-C₄-Alkyl- oder Phenylgruppe steht; 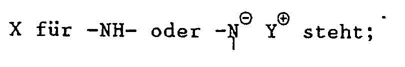 worin
Y⊕ ein anorganisches oder organisches Kation repräsentiert;
R₆ Wasserstoff oder C₁-C₆-Alkyl bedeutet; und
R₇ für ein unsubstituiertes oder substituiertes Phenyl, Naphthyl, Pyridyl, Pyridazinyl, Pyrimidinyl oder Pyrazinyl steht, wobei die Substituenten ausgewählt sind aus der Reihe Halogen, Alkyl, Halogenalkyl, Alkoxy, Halogenalkoxy, Alkylthio, Halogenalkylthio, Alkenyl, Halogenalkenyl, Alkinyl, Halogenalkinyl, Alkylsulfinyl, Alkylsulfonyl, Halogenalkylsulfinyl, Halogenalkylsulfonyl, Alkylamino, Dialkylamino und Benzyl sowie durch Substituenten der Gruppe bestehend aus Halogen, Halogenalkyl, Halogenalkoxy und Nitro substituiertes Phenoxy oder Pyridyloxy, wobei, wenn R₇ für substituiertes Phenyl steht, als Substituent auch Cyan, N'- n-Propyl-N'-phenylureido, eine Brücke -O-CF₂-CF₂-O-, welche zwei benachbarte Kohlenstoffatome des Phenylrings miteinander verbindet, oder Phenoxy, welches durch eine Brücke -O-CF₂-CF₂-O- substituiert ist, welche zwei benachbarte Kohlenstoffatome des Phenylrings miteinander verbindet, vorliegen können.

3. Mittel nach Anspruch 2, dadurch gekennzeichnet, dass
R₁, R₂, R₃ und R₅ unabhängig voneinander für Wasserstoff, Halogen, C₁-C₆-Alkyl, Ci-C₆-Haloalkyl, C₁-C₆-Alkoxy, C₁-C₆-Haloalkoxy oder C₁-C₆-Alkylthio stehen;
R₄ für Wasserstoff, Halogen, C₁-C₆-Alkyl, C₁-C₆-Haloalkyl, C₁-C₆-Alkoxy, C₁-C₆-Haloalkoxy; C₁-C₆-Alkylthio oder NHR' steht; worin R' Wasserstoff, R₈CO- oder R₉NHCO- bedeutet, wobei R₈ für ein C₁-C₄-Alkyl steht, welches unsubstituiert oder mit einem bis drei gleichen oder verschiedenen Substituenten aus der Gruppe Halogen, Ci-C₄-Alkoxy, C₁-C₄-Acyloxy und -COOG substituiert ist, worin G Wasserstoff, ein Alkali- oder Erdalkalimetallkation bedeutet, und R₉ für eine unsubstituierte oder ein- bis dreifach durch Halogen substituierte C₁-C₄-Alkyl- oder Phenylgruppe steht; 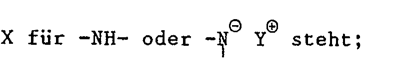
worin
Y⊕ ein anorganisches oder organisches Kation repräsentiert;
R₆ Wasserstoff oder C₁-C₆-Alkyl bedeutet; und
R₇ für ein unsubstituiertes oder substituiertes Phenyl, Naphthyl, Pyridyl, Pyridazinyl, Pyrimidinyl oder Pyrazinyl steht, wobei die Substituenten ausgewählt sind aus der Reihe Halogen, Alkyl, Halogenalkyl, Alkoxy, Halogenalkoxy, Alkylthio, Halogenalkylthio, Alkenyl, Halogenalkenyl, Alkinyl, Halogenalkinyl, Alkylsulfinyl, Alkylsulfonyl, Halogenalkylsulfinyl, Halogenalkylsulfonyl, Alkylamino, Dialkylamino oder Benzyl sowie durch Halogen, Halogenalkyl, Halogenalkoxy oder Nitro substituiertes Phenoxy oder Pyridyloxy.
4. Mittel nach Anspruch 2, dadurch gekennzeichnet, dass R₁ und R₅ unabhängig voneinander für Wasserstoff, Fluor, Chlor, Methoxy oder Methylthio stehen;
R₃ Wasserstoff oder Fluor und R₄ Wasserstoff oder NH₂ repräsentieren;
R₂ für Wasserstoff, Fluor oder Chlor steht; 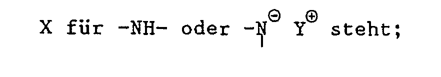
worin
Y⊕ für Na⊕, K^{⊕} oder Tetraalkylammonium, wie (n-C₄H₉)₄N^{⊕}, (CH₃)₄N^{⊕}, (C₂H₅)₄N^{⊕} oder n-C₁₆H₃₃-N^{⊕}(CH₃)₃ steht;
R₆ für Wasserstoff oder Ci-C₃-Alkyl steht und
R₇ für unsubstituiertes oder durch ein bis zwei Halogenatome und zusätzlich durch C₁-C₆-Halogenalkoxy oder 2-Pyridyloxy substituiertes Phenyl steht, wobei der 2-Pyridyloxyrest seinerseits durch CF₃ und Halogen substituiert ist.

5. Mittel nach Anspruch 1, dadurch gekennzeichnet, dass es Verbindungen der Formel II 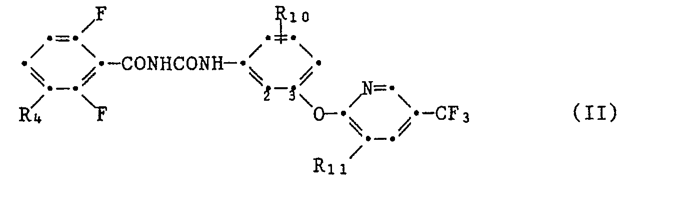 worin
R₄ Wasserstoff oder NH₂ bedeutet;
R₁₀ für Wasserstoff, Halogen oder Methyl steht; und
R₁₁ Wasserstoff oder Halogen bedeutet, enthält.
6. Mittel nach Anspruch 5, dadurch gekennzeichnet, dass
R₄ Wasserstoff oder NH₂ bedeutet;
R₁₀ für Wasserstoff, Fluor, Chlor oder Brom steht; und
R₁₁ Wasserstoff, Fluor, Chlor oder Brom bedeutet.
7. Mittel nach Anspruch 5, dadurch gekennzeichnet, dass
R4 Wasserstoff oder NH₂ bedeutet;
R₁₀ für 4-Fluor, 4-Chlor, 4-Brom oder 4-Methyl steht; und
R₁₁ i Chlor bedeutet.

8. Mittel nach Anspruch 1, dadurch gekennzeichnet, dass es N-[3-(3-Chlor-5-trifluormethylpyridyl-2-oxy)-4- chlorphenyl]-N'-[2,6-difluorbenzoyl]-harnstoff als Wirkstoff enthält.

9. Mittel nach Anspruch 1, dadurch gekennzeichnet, dass es N-[3-(3-Chlor-5-trifluormethylpyridyl-2-oxy)-4- chlorphenyl]-N'-[2,6-difluor-3-aminobenzoyl]-harnstoff als Wirkstoff enthält.

10. Mittel nach Anspruch 1, dadurch gekennzeichnet, dass es sich bei dem 1-substituierten Azacycloalkan-2-on um eine Verbindung der Formel III 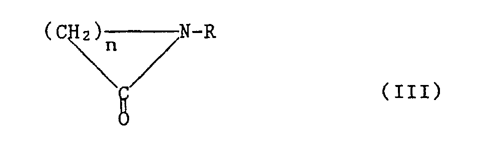 handelt, wobei n eine ganze Zahl von 2 bis 7 bedeutet und R für ein C6-Cls-Alkyl steht, das gegebenenfalls durch ein Sauerstoffatom unterbrochen ist.

11. Mittel nach Anspruch 1, dadurch gekennzeichnet, dass es n-Dodecylazacycloheptan-2-on enthält.

12. Mittel nach Anspruch 1, dadurch gekennzeichnet, dass es n-Dodecylazacyclopentan-2-on enthält.

13. Mittel nach Anspruch 8, dadurch gekennzeichnet, dass es n-Dodecylazacycloheptan-2-on enthält.

14. Mittel nach Anspruch 8, dadurch gekennzeichnet, dass es n-Dodecylazacyclopentan-2-on enthält.

15. Mittel nach Anspruch 1, dadurch gekennzeichnet, dass es als hydrophiles Lösungsmittel Dimethylsulfoxid enthält.

16. Mittel nach Anspruch 1, dadurch gekennzeichnet, dass es als hydrophiles Lösungsmittel N-Methylpyrrolidon enthält.

17. Mittel nach Anspruch 1, dadurch gekennzeichnet, dass es ein lipophiles Lösungsmittel der Gruppe bestehend aus Estern von Carbonsäuren sowie flüssigen Wachsen enthält.

18. Mittel nach Anspruch 1, dadurch gekennzeichnet, dass es als stabilisierende Komponente eine Säure, ausgewählt aus der Gruppe bestehend aus Zitronensäure, Ascorbinsäure, Milchsäure, Aepfelsäure und Weinsäure, enthält.

19. Verfahren zur Herstellung eines Mittels zur Bekämpfung von Parasiten, die Nutz- und Haustiere befallen, das parenteral injizierbar ist und 0,1 bis 10 % eines Benzoylphenylharnstoffes als Wirkstoff, 0,1 bis 60 % eines 1-substituierten Azacycloalkan-2-ons, 2 bis 90 % eines physiologisch verträglichen Tensids oder Tensidgemisches und gegebenenfalls als stabilisierende Komponente 0,05 bis 1 % einer Säure oder eines Puffergemisches sowie ad 100 % eines physiologisch verträglichen hydrophilen Lösungsmittels oder Lösungsmittelgemisches oder ein Gemisch von physiologisch verträglichen hydrophilen und lipophilen Lösungsmitteln enthält, dadurch gekennzeichnet, dass der Benzoylphenylharnstoff in einem hydrophilen Lösungsmittel gelöst und mit dem Tensid sowie dem 1-substituierten Azacycloalkan-2-on, gegebenenfalls dem lipophilen Lösungsmittel und gegebenenfalls einem Stabilisator vermischt, mit einem Lösungsmittel aufgefüllt und die so erhaltene Endmischung durch ein Filter sterilfiltriert und anschliessend hitzesterilisiert wird.

20. Mittel nach Anspruch 1 zur Verwendung in einem Verfahren zur Bekämpfung von Parasiten, welche Haus- und Nutztiere befallen.

Patentansprüche für folgende Vertragsstaaten: ES, GR

1. Verfahren zur Herstellung eines Mittels zur Bekämpfung von Parasiten, die Nutz- und Haustiere befallen, das parenteral injizierbar ist und 0,1 bis 10 % eines Benzoylphenylharnstoffes als Wirkstoff, 0,1 bis 60 % eines 1-substituierten Azacycloalkan-2-ons, 2 bis 90 % eines physiologisch verträglichen Tensids oder Tensidgemisches und gegebenenfalls als stabilisierende Komponente 0,05 bis 1 % einer Säure oder eines Puffergemisches sowie ad 100 % eines physiologisch verträglichen hydrophilen Lösungsmittels oder Lösungsmittelgemisches oder ein Gemisch von physiologisch verträglichen hydrophilen und lipophilen Lösungsmitteln enthält, dadurch gekennzeichnet, dass der Benzoylphenylharnstoff in einem hydrophilen Lösungsmittel gelöst und mit dem Tensid sowie dem 1-substituierten Azacycloalkan-2-on, gegebenenfalls dem lipophilen Lösungsmittel und gegebenenfalls einem Stabilisator vermischt, mit einem Lösungsmittel aufgefüllt und die so erhaltene Endmischung durch ein Filter sterilfiltriert und anschliessend hitzesterilisiert wird.

2. Verfahren zur Herstellung eines Mittels nach Anspruch 1, welches Verbindungen der Formel I 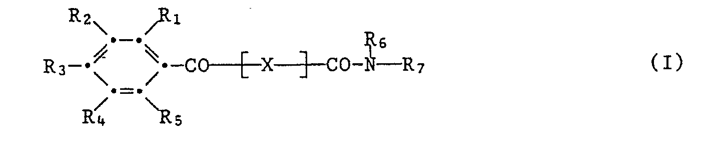 enthält, worin
R₁, R₂, R₃ und R₅ unabhängig voneinander für Wasserstoff, Halogen, Ci-C₆-Alkyl, Ci-C₆-Haloalkyl, C₁-C₆-Alkoxy, C₁-C₆-Haloalkoxy oder C₁-C₆-Alkylthio stehen;
R₄ für Wasserstoff, Halogen, C₁-C₆-Alkyl, C₁-C₆-Haloalkyl, C₁-C₆-Alkoxy, C₁-C₆-Haloalkoxy; C₁-C₆-Alkylthio oder NHR' steht; worin R' Wasserstoff, R₈CO- oder RsNHCO- bedeutet, wobei R₈ für ein C₁-C₄-Alkyl steht, welches unsubstituiert oder mit einem bis drei gleichen oder verschiedenen Substituenten aus der Gruppe Halogen, C₁-C₄-Alkoxy, C₁-C₄-Acyloxy und -COOG substituiert ist, worin G Wasserstoff, ein Alkali- oder Erdalkali metallkation bedeutet, und R₉ für eine unsubstituierte oder ein- bis dreifach durch Halogen substituierte C₁-C₄-Alkyl- oder Phenylgruppe steht; 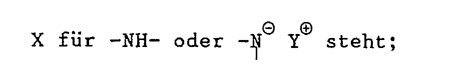
worin
Y^{⊕} ein anorganisches oder organisches Kation repräsentiert;
R₆ Wasserstoff oder C₁-C₆-Alkyl bedeutet; und
R₇ für ein unsubstituiertes oder substituiertes Phenyl, Naphthyl, Pyridyl, Pyridazinyl, Pyrimidinyl oder Pyrazinyl steht, wobei die Substituenten ausgewählt sind aus der Reihe Halogen, Alkyl, Halogenalkyl, Alkoxy, Halogenalkoxy, Alkylthio, Halogenalkylthio, Alkenyl, Halogenalkenyl, Alkinyl, Halogenalkinyl, Alkylsulfinyl, Alkylsulfonyl, Halogenalkylsulfinyl, Halogenalkylsulfonyl, Alkylamino, Dialkylamino und Benzyl sowie durch Substituenten der Gruppe bestehend aus Halogen, Halogenalkyl, Halogenalkoxy und Nitro substituiertes Phenoxy oder Pyridyloxy, wobei, wenn R₇ für substituiertes Phenyl steht, als Substituent auch Cyan, N'- n-Propyl-N'-phenylureido, eine Brücke -O-CF₂-CF₂-0-, welche zwei benachbarte Kohlenstoffatome des Phenylrings miteinander verbindet, oder Phenoxy, welches durch eine Brücke -O-CF₂-CF₂-O- substituiert ist, welche zwei benachbarte Kohlenstoffatome des Phenylrings miteinander verbindet, vorliegen können.

3. Verfahren zur Herstellung eines Mittels nach Anspruch 2, welches Verbindungen der Formel I enthält, worin
R₁, R₂, R₃ und R₅ unabhängig voneinander für Wasserstoff, Halogen, C₁-C₆-Alkyl, C₁-C₆-Haloalkyl, C₁-C₆-Alkoxy, C₁-C₆-Haloalkoxy oder C₁-C₆-Alkylthio stehen;
R₄ für Wasserstoff, Halogen, C₁-C₆-Alkyl, C₁-C₆-Haloalkyl, C₁-C₆-Alkoxy, C₁-C₆-Haloalkoxy; C₁-C₆-Alkylthio oder NHR' steht; worin R Wasserstoff, R₈CO- oder RsNHCO- bedeutet, wobei R₈ für ein C₁-C₄-Alkyl steht, welches unsubstituiert oder mit einem bis drei gleichen oder verschiedenen Substituenten aus der Gruppe Halogen, C₁-C₄-Alkoxy, C₁-C₄-Acyloxy und -COOG substituiert ist, worin G Wasserstoff, ein Alkali- oder Erdalkalimetallkation bedeutet, und Rs für eine unsubstituierte oder ein- bis dreifach durch Halogen substituierte C₁-C₄-Alkyl- oder Phenylgruppe steht; 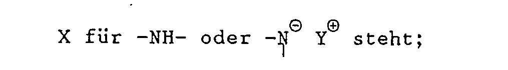
worin
Y^{⊕} ein anorganisches oder organisches Kation repräsentiert;
R₆ Wasserstoff oder Cᵢ-C₆-Alkyl bedeutet; und
R₇ für ein unsubstituiertes oder substituiertes Phenyl, Naphthyl, Pyridyl, Pyridazinyl, Pyrimidinyl oder Pyrazinyl steht, wobei die Substituenten ausgewählt sind aus der Reihe Halogen, Alkyl, Halogenalkyl, Alkoxy, Halogenalkoxy, Alkylthio, Halogenalkylthio, Alkenyl, Halogenalkenyl, Alkinyl, Halogenalkinyl, Alkylsulfinyl, Alkylsulfonyl, Halogenalkylsulfinyl, Halogenalkylsulfonyl, Alkylamino, Dialkylamino oder Benzyl sowie durch Halogen, Halogenalkyl, Halogenalkoxy oder Nitro substituiertes Phenoxy oder Pyridyloxy.

4. Verfahren zur Herstellung eines Mittels nach Anspruch 2, welches Verbindungen der Formel I enthält, worin R₁ und R₅ unabhängig voneinander für Wasserstoff, Fluor, Chlor, Methoxy oder Methylthio stehen; R₃ Wasserstoff oder Fluor und R₄ Wasserstoff oder NH₂ repräsentieren; R₂ für Wasserstoff, Fluor oder Chlor steht; 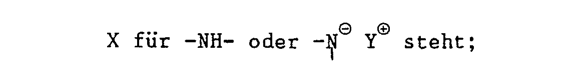 worin
Y^{⊕} für Na^{⊕}, K^{⊕} oder Tetraalkylammonium, wie (n-C₄H₉)₄N^{⊕}, (CH₃)₄N^{⊕}, (C₂H₅)₄N^{⊕} oder n-C₁₆H₃₃-N^{⊕}(CH₃)₃ steht;
R₆ für Wasserstoff oder C1-C3-Alkyl steht und
R₇ für unsubstituiertes oder durch ein bis zwei Halogenatome und zusätzlich durch C₁-C₆-Halogenalkoxy oder 2-Pyridyloxy substituiertes Phenyl steht, wobei der 2-Pyridyloxyrest seinerseits durch CF₃ und Halogen substituiert ist.

5. Verfahren zur Herstellung eines Mittels nach Anspruch 1, welches Verbindungen der Formel II 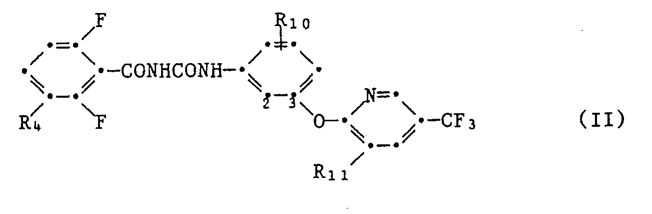 enthält, worin
R₄ Wasserstoff oder NH₂ bedeutet;
R₁₀ für Wasserstoff, Halogen oder Methyl steht; und
R_{"} Wasserstoff oder Halogen bedeutet.

6. Verfahren zur Herstellung eines Mittels nach Anspruch 5, welches Verbindungen der Formel II enthält, worin
R₄ Wasserstoff oder NH₂ bedeutet;
R₁₀ für Wasserstoff, Fluor, Chlor oder Brom steht; und
R₁₁ Wasserstoff, Fluor, Chlor oder Brom bedeutet.

7. Verfahren zur Herstellung eines Mittels nach Anspruch 5, welches Verbindungen der Formel II enthält, worin
R₄ Wasserstoff oder NH₂ bedeutet;
R₁₀ für 4-Fluor, 4-Chlor, 4-Brom oder 4-Methyl steht; und
R_{"} Chlor bedeutet.

8. Verfahren zur Herstellung eines Mittels nach Anspruch 1, welches N-[3-(3-Chlor-5-trifluormethylpyridyl-2- oxy)-4-chlorphenyl]-N'-[2,6-difluorbenzoyl]-harnstoff als Wirkstoff enthält.

9. Verfahren zur Herstellung eines Mittels nach Anspruch 1, welches N-[3-(3-Chlor-5-trifluormethylpyridyl-2- oxy)-4-chlorphenyl]-N'-[2,6-difluor-3-aminobenzoyl]-harnstoff als Wirkstoff enthält.

10. Verfahren zur Herstellung eines Mittels nach Anspruch 1, welches als 1-substituiertes Azacycloalkan-2- on eine Verbindung der Formel III 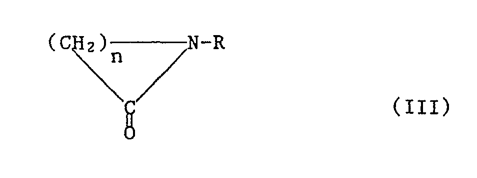 enthält, wobei n eine ganze Zahl von 2 bis 7 bedeutet und R für ein C₆-C₁₅-Alkyl steht, das gegebenenfalls durch ein Sauerstoffatom unterbrochen ist.

11. Verfahren zur Herstellung eines Mittels nach Anspruch 1, welches n-Dodecylazacycloheptan-2-on enthält.

12. Verfahren zur Herstellung eines Mittels nach Anspruch 1, welches n-Dodecylazacyclopentan-2-on enthält.

13. Verfahren zur Herstellung eines Mittels nach Anspruch 8, welches n-Dodecylazacycloheptan-2-on enthält.

14. Verfahren zur Herstellung eines Mittels nach Anspruch 8, welches n-Dodecylazacyclopentan-2-on enthält.

15. Verfahren zur Herstellung eines Mittels nach Anspruch 1, welches als hydrophiles Lösungsmittel Dimethylsulfoxid enthält.

16. Verfahren zur Herstellung eines Mittels nach Anspruch 1, welches als hydrophiles Lösungsmittel N-Methyl-pyrrolidon enthält.

17. Verfahren zur Herstellung eines Mittels nach Anspruch 1, welches ein lipophiles Lösungsmittel der Gruppe bestehend aus Estern von Carbonsäuren sowie flüssigen Wachsen enthält.

18. Verfahren zur Herstellung eines Mittels nach Anspruch 1, welches als stabilisierende Komponente eine Säure, ausgewählt aus der Gruppe bestehend aus Zitronensäure, Ascorbinsäure, Milchsäure, Aepfelsäure und Weinsäure, enthält.
